# EUROPÄISCHE PATENTSCHRIFT

(11) **EP 2 531 229 B1**
(45) Veröffentlichungstag und Bekanntmachung des Hinweises auf die Patenterteilung: **31.12.2014**
(21) Anmeldenummer: 11738588.0
(22) Anmeldetag: 27.05.2011
(51) Int. Cl.: A61L 29/08, A61L 29/16, A61L 31/10, A61L 31/16

(54) **MEDIZINPRODUKT MIT EINER PARTIKELFREIEN WIRKSTOFF-ABGEBENDEN BESCHICHTUNG**
MEDICINE PRODUCT WITH A PARTICLE-FREE COATING RELEASING AN ACTIVE SUBSTANCE
PRODUIT MÉDICAL REVÊTU D'UNE COUCHE NON PARTICULAIRE DE LIBÉRATION DE PRINCIPE ACTIF

(30) Priorität: 27.05.2010 DE 102010022588
(43) Veröffentlichungstag der Anmeldung: 12.12.2012
(73) Patentinhaber: Hemoteq AG, 52146 Würselen (DE)
(72) Erfinder: HOFFMANN, Erika, 52249 Eschweiler (DE); HOFFMANN, Michael, 52249 Eschweiler (DE); HORRES, Roland, 52223 Stolberg (DE)
(74) Vertreter: Arth, Hans-Lothar
(86) Internationale Anmeldenummer: PCT/DE2011/001151
(87) Internationale Veröffentlichungsnummer: WO 2011/147408

(56) Entgegenhaltungen:
- WO-A2-2008/086794
- US-A1- 2008 118 544
- US-A1- 2010 055 294

## Beschreibung

Die vorliegende Erfindung betrifft mit dem Organismus in Kontakt kommende Medizinprodukte wie beispielsweise Kurzzeitimplantate und Langzeitimplatate, beschichtet mit mindestens einer Schicht, welche einen molekulardispers verteilten bzw. gelösten Wirkstoff in mindestens einer Trägersubstanz und optional einem oder mehreren Hilfsstoffen enthält, wobei diese mindestens eine Schicht eine streichfeste Lösung bildet, Verfahren zur Herstellung dieser Beschichtung aus streichfesten Lösungen und die Verwendung der mit einer streichfesten Lösung beschichteten Medizinprodukte.

Die Verbesserungen, die in der zurückliegenden Zeit den Ballonkathetern widerfahren sind, bezogen und beziehen sich bislang vorrangig auf ihre Fähigkeiten, einen Stent präzise und sicher zu platzieren. Die PTCA als eigenständige Methode wurde - und wird immer noch - vorrangig zur Entfernung von bestehenden Verengungen durch kurzzeitige Dilatation und damit Weitung des Gefässes eingesetzt, wurde aber von der Stentimplantation als bleibende Stütze besonders im koronaren Bereich weitgehend verdrängt. Die Nutzung der PTCA zur Restenoseprophylaxe steckt daher immer noch in den Kinderschuhen.

Allerdings bestehen bei der Anwendung der PTCA klare Vorteile gegenüber dem Stent, nicht zuletzt deshalb, weil sich auf diese Weise zu keinem Zeitpunkt nach Durchführung der Behandlung ein Fremdkörper als zusätzliche Belastung bzw. Initiator für Folgeerscheinungen wie sie auch die Restenose ist, im Organismus befindet. Deshalb gibt es Anknüpfungen an die Ende der 80er Jahre durchgeführten Arbeiten eines wirkstofffreisetzenden Ballonkatheters.

So wurden beispielsweise verschiedenartige Ausführungsformen von Ballonkathetern beschrieben, deren mit der Umgebung in direktem Kontakt stehende Hülle über Löcher verfügt, durch die während der Dilatation unter Druck ein gelöster bzw. flüssiger Wirkstoff an die Gefäßwand gedrückt wird (z.B. in US5087244, US4994033, US4186745). EP 0 383 429 A offenbart beispielsweise einen Ballonkatheter mit winzigen Löchern, durch die bei der Dilatation eine Heparinlösung an die Gefäßwand abgegeben wird.

Von den wenigen aktuell auf dem Markt erhältlichen wirkstoffabgebenden Ballonkathetern (DEB) haben die einen den Nachteil, dass sie den Wirkstoff Paclitaxel zwar als Beschichtung auf dem Ballon enthalten, ihn bei der Dilatation aber nicht abgeben und daher nicht wirksamer sind als unbeschichtete Ballonkatheter. Die anderen haben den von der FDA und den europäischen Zulassungsstellen bemängelten Nachteil, dass sie bei der Dilatation den Wirkstoff Paclitaxel in Form von schwerlöslichen Partikeln abgeben, die das Risiko von Gefässverschlüssen distal von der Dilatationsstelle verursachen und damit eine erhebliche Gefährdung des Patienten darstellen.

An diesen Beispielen erkennt man die Schwierigkeiten, die mit der Anwendbarkeit der effektiv wirksamen Wirkstoffe vor allem in der PTCA entstehen. Da die Wirkfähigkeit dieser Wirkstoffe nicht nur von ihren chemischen und physiologischen Eigenschaften, sondern auch von ihren physikalischen Eigenschaften determiniert wird, sind ebenso sehr physikalische Faktoren wie Aggregatzustand, Löslichkeit, Viskosität, Dichte, Siede- bzw. Schmelzpunkt zu berücksichtigen. Diese sind maßgeblich für die Verarbeitung und Praktikabilität der Substanz verantwortlich und bestimmen die Anwendungsmöglichkeiten im erheblichen Maße mit. Folglich ist die erfolgreiche Verwendung eines Wirkstoffes an seine Verarbeitbarkeit und erzielbare Verfügbarkeit am Zielort geknüpft.

Als ein Beispiel sei auch hier das stark lipophile und wasserunlösliche Chemotherapeutikum Paclitaxel genannt, das in Form einer Emulsion als Injektionslösung bzw. Infusionslösung angewendet wird. Als Lösungsmittel wird Ethanol (mit ca. 50 Vol%) und eine Mischung aus Macrogolricinoleat (Cremophor EL) als Emulgator verwendet, das die Löslichkeit im wässrigen System Blut erhöht. Es ist erwiesen, dass der Zusatz von Cremophor EL für diverse, teilweise schwere Akzeptanzprobleme mitverantwortlich ist (New Engl J of Medicine, Vol 332 Nr 15, 1995*: 1004*). Nichtsdestotrotz ist Cremophor ein notwendiges und deshalb oft eingesetztes Additiv zur Erhöhung der Löslichkeit wasserunlöslicher Wirkstoffe wie Paclitaxel, Cyclosporin, Vitamin K, Propofol, Diazepam, etc.. Um die Anwendungen von Paclitaxel auf wasserlösliche Systeme zu erweitern, wurde durch die Herstellung von Paclitaxelderivaten z.B. durch kovalente Bindung an wasserlösliche Polymere versucht, die Verfügbarkeit ohne Verlust der Wirkfähigkeit im Gefäßsystem des Organismus zu erhöhen (US5648506A; US6262107B1, US6441025B2), aber der Erfolg blieb aus.

Gerade die antiproliferativen Wirkstoffe Paclitaxel und Rapamycin haben sich nicht nur in der Antitumortherapie, sondern allgemein als erfolgreiches Mittel gegen übermässiges Zellwachstum erwiesen. Hierzu werden die Wirkstoffe entweder systemisch oder mit Hilfe eines Medizinproduktes ohne polymere Matrix oder in einer festen und vorzugsweise polymeren Matrix eingelagert in direktem Kontakt mit der zu behandelnden Stelle im Organismus gebracht. Der Vorteil für die nicht systemische Anwendung liegt auf der Hand, da die applizierten Wirkstofffmengen mittels Medizinprodukt so gering sind, dass die toxischen Nebenwirkungen, die man von systemischer Gabe kennt, nicht eintreten und es für den Organismus zu keiner Belastung kommt.

Aber genau die nicht systemische und wesentlich schonendere direkte Behandlung erkrankter Stellen und insbesondere verengter Stellen von Blutgefäßen mit hydrophoben und effektiven Wirkstoffen ergeben aufgrund der physikalischen Eigenschaften Handhabungs- und Verfügbarkeitsengpässe bzw. massive Einschränkungen.

Somit bedarf es besonderer Beschichtungen, mit welchen bereits auch in relativ kurzer Zeit kontrolliert und therapeutisch ausreichende Mengen Wirkstoff in die Zelle eingebracht werden können.

Wirkstoffe wie Paclitaxel und Rapamycin haben sich als besonders geeignet gegen hyperproliferative Zellen herausgestellt. Gerade diese hochwirksamen Wirkstoffe besitzen die oben beschriebenen Nachteile hinsichtlich der Partikelfreisetzung bei der Dilatation und der Wasserunlöslichkeit. Daher hat sich unter anderem die Aufgabe gestellt, wirksame Substanzen so auf einen Katheterballon aufzubringen, dass eine hydrophile Beschichtung entsteht, welche sich während der Dilatation des Gefässes leicht von dem Ballon ablöst, den Wirkstoff in kolloidaler oder gelöster Form freisetzt und effektiv und vor allem ohne die Entstehung von Partikeln auf die Gefäßwand überträgt.

Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Die vorliegende Erfindung betrifft medizinische Kurtzzeitimplanat und/oder Langzeitimplanat, wobei die Oberfläche der Vorrichtung vollständig oder teilweise mit einer streichfesten Lösung enthaltend mindestens einen Wirkstoff und mindestens einer Trägersubstanz beschichtet ist.

Bei den mit dem Organismus in Kontakt kommenden Medizinprodukten oder medizinischen Vorrichtungen, wie hierin beschrieben, handelt es sich um Implantate, welche dauerhaft im Körper verbleiben wie Stents und Gefäßprothesen und daher hierin als Langzeitimplantate bezeichnet werden, aber auch wieder entfernbare über einen begrenzten Zeitraum eingesetzte Vorrichtungen wie Katheter, z.B. Blasenkatheter, Beatmungsschläuche, Venenkatheter, Ballonkatheter, Dilatationskatheter, PTCA- und PTA-Katheter, Embolektomiekatheter, Valvuloplastiekatheter und Kanülen. Bei den vorgenannten Kathetern, wird der Ballon, der Katheterballon oder der dilatierbare Bereich des Katheters mit der Beschichtung in Form einer streichfesten Lösung versehen.

Es ist bevorzugt, wenn die Beschichtung keine Suspension und keine feste Dispersion ist, da feste Dispersionen zu brüchig sind und mit hoher Wahrscheinlichkeit während der Implantation oder der Einführung des Katheterballons abplatzen. Eine besonders große Gefahr stellt eine brüchige oder spröde Beschichtung dar, die auf expandierbare, medizinische Vorrichtungen aufgebracht wird. Dies ist vor allem bei Implantaten in Hohlorganen (Stents, Ballonkatheter) schädlich, da die Bruchstücke hier zu einer Okklusion führen können. Besonders im Blutgefäßsystem müssen Bruchstücke der Beschichtung verhindert werden, da hier in den immer kleiner werdenden Gefäßen bereits kleinste Bruchstücke die Gefahr einer Stenose und einer sofortigen Schädigung des entsprechenden Gewebes bis hin zum Herzinfarkt mit sich bringen. Bei diesen Bruchstücken kann es sich um kristalline Partikel des Wirkstoffs als auch um Partikel einer festen Beschichtung handeln, welche sich bei der Einführung des Katheters und/oder bei der Dilatation des Katheterballons ablösen.

Der Begriff feste Dispersion beschreibt Systeme im festen Zustand, die mindestens einen Wirkstoff in einer inerten Trägersubstanz enthalten. Unter den Oberbegriff feste Dispersion fallen sowohl feste Suspensionen als auch feste Lösungen. Bei einer festen Suspension liegt der Wirkstoff partikulär im Träger vor. Dagegen ist der Wirkstoff bei festen Lösungen molekulardispers verteilt, d.h. er ist in der Grundstruktur des Trägers gelöst.

Bei festen Lösungen fungiert ein im festen Zustand vorliegender Träger als festes Lösungsmittel für die Wirkstoffmoleküle. Bei kristallinen festen Lösungen ist der Wirkstoff entweder in das Kristallgitter eingebaut oder befindet sich im Raum zwischen den Gittermolekülen des Trägers. Ist der Träger mit den gelösten Wirkstoffmolekülen dagegen amorph, spricht man von glasartigen festen Lösungen.

Der Begriff "streichfeste Lösung" wie hierin verwendet, bezeichnet ein System aus mindestens einem Wirkstoff, der molekulardispers in einer zähflüssigen Trägersubstanz vorliegt, die kein Feststoff sondern eine Flüssigkeit ist. Wie bei jeder Lösung sind die Wirkstoffmoleküle in der Trägersubstanz gelöst. Die streichfeste Lösung wie hierin definiert ist nicht mehr fließfähig. Der Begriff "molekulardispers" wie hierin verwendet, besagt, dass der Wirkstoff im Träger in einzelnen Molekülen vorliegt, die im Träger verteilt sind, d.h. die Teilchengröße des Wirkstoffs liegt im Bereich eines einzelnen Wirkstoffmoleküls.

Insbesondere von dem Wirkstoff Paclitaxel ist bekannt, dass größere Partikel bestehend aus oder enthaltend den Wirkstoff Paclitaxel deutlich weniger wirksam bis unwirksam zur Restenoseprophylaxe im Vergleich zu sehr feinen Wirkstoffkristallen sind. Nach Erkenntnissen der Anmelderin scheint sich diese Aussage auch auf Rapamycin und auf andere Indikationen als Restenose übertragen zu lassen.

Die streichfeste Lösung bzw. die Beschichtung auf der medizinischen Vorrichtung, dem Stent, dem Katheterballon ohne Stent oder dem Katheterballon mit Stent hat bevorzugt eine dynamische Viskosität zwischen 10⁵ mPa·s und 10¹⁸ mPa·s, weiter bevorzugt zwischen 1,5·10⁵ mPa·s und 10¹³ mPa·s, noch weiter bevorzugt zwischen 2,5·10⁵ mPa·s und 10¹² mPa·s und besonders bevorzugt zwischen 10⁶ mPa·s und 10⁹ mPa·s. Die streichfeste Lösung bzw. die Beschichtung auf der erfindungsgemäßen Vorrichtung hat also bevorzugt eine Viskosität > 250 Pa·s und besonders bevorzugt > 1000 Pa·s. Alle hier genannten Werte beziehen sich auf die Viskosität bei 20 °C.

Die Viskosität ist ein Maß für die Zähflüssigkeit einer Flüssigkeit oder eines Gases. Der Kehrwert der Viskosität ist die Fluidität, ein Maß für die Fließfähigkeit einer Flüssigkeit oder einer Lösung. Je größer die Viskosität, desto dickflüssiger (weniger fließfähig) ist die Flüssigkeit; je niedriger die Viskosität, desto dünnflüssiger (fließfähiger) ist sie.

Normalerweise wird mit dem Begriff Viskosität die Viskosität in Scherung verbunden, es ist allerdings auch möglich, die Viskosität in Dehnung zu messen. Der Fachmann weiß, dass es dazu verschiedene Arten von Viskosimetern, Messgeräte zur Bestimmung der Zähigkeit (Viskosität) einer Flüssigkeit, gibt, deren Anwendung zum Allgemeinwissen eines Fachmanns gehört.

Festkörper befinden sich im festen Aggregatzustand. Besonderes Kennzeichen von Festkörpern ist die Beständigkeit der Ordnung (amorph oder kristallin) ihrer Bausteine. Eine Flüssigkeit ist Materie im flüssigen Aggregatzustand. Eine Flüssigkeit ist ein Stoff, der einer Formänderung so gut wie keinen, einer Volumenänderung hingegen einen recht großen Widerstand entgegensetzt. Flüssigkeiten sind also volumenbeständig, formunbeständig und unterliegen einer ständigen Brownschen Bewegung.

Der Begriff "streichfeste Lösung" wie hierin verwendet, soll unterkühlte Schmelzen umfassen. Eine unterkühlte Schmelze stellt einen Zustand dar, in dem ein Stoff in einem Aggregatzustand verbleibt, der unterhalb des Umwandlungspunktes eigentlich nicht vorliegen sollte. Eine unterkühlte Schmelze oder Flüsigkeit hat bei gegebenem Druck eine niedrigere Temperatur, als ihrem Aggregatzustand entspricht.

Geht man vom flüssigen Zustand eines Stoffes oberhalb seiner Schmelztemperatur Tm aus (Schmelze), so findet beim Unterschreiten von Tm normalerweise Kristallisation des Stoffes statt. Kommt es dagegen beim Abkühlen der Schmelze zu keiner Kristallisation nach Unterschreiten von Tm, so entsteht eine sogenannte unterkühlte Schmelze. In diesem Fall ist entweder der Abkühlprozess aufgrund einer hohen Kühlrate zu schnell für den Kristallisationsprozess, oder die Moleküle des betrachteten Stoffes zeigen ungünstige Eigenschaften für eine schnelle Kristallisation. Bei der Unterschreitung der Tm ohne eine Kristallisation kommt es zu keiner sprunghaften Änderung der Enthalpie oder des freien Volumens, wie dies bei einem Phasenübergang erster Ordnung der Fall ist.

Am Glasübergang ist die molekulare Beweglichkeit der unterkühlten Schmelze soweit reduziert, dass bei einer weitereren Temperaturerniedrigung der Gleichgewichtszustand der unterkühlten Schmelze nicht so schnell erreicht werden kann, wie dem System thermische Energie entzogen wird. Die Moleküle der unterkühlten Flüssigkeit werden beim Glasübergang kinetisch eingefroren und haben deshalb eine ähnliche Zufallsanordnung wie im flüssigen Zustand. Im Gegensatz zur unterkühlten Schmelze ist die molekulare Beweglichkeit im glasartigen Zustand jedoch wesentlich geringer.

Der Zustand der unterkühlten Schmelze (gummielastischer Zustand) und der glasartige Zustand einer Substanz werden durch die Glasübergangstemperatur Tg getrennt. Oberhalb dieser Temperatur liegt die Substanz als unterkühlte Schmelze vor und unterhalb befindet sich die Substanz im glasartig festen Zustand, der sich durch eine Viskosität größer als 10¹² Pa·s auszeichnet.

Bei der Glasübergangstemperatur Tg ändert sich der Wert der Wärmekapazität cₚ sprunghaft. Im glasartigen Zustand, das heißt unterhalb der Tg ist der Wert der Wärmekapazität deutlich niedriger und der Verlust an thermischer Energie erfolgt bei weiter sinkenden Temperaturen deutlich langsamer. Der Übergang von einer unterkühlten Schmelze zu einer glasartigen festen Lösung ist also durch eine sprunghafte Änderung der Wärmekapazität gekennzeichnet. Es kommt beim Übergang in den glasartigen Zustand nicht zu einer Enthalpieänderung. Dem Fachmann ist bekannt, wie die Glasübergangstemperatur Tg bestimmt werden kann, so dass durch Bestimmung der Glasübergangstemperatur Tg die erfindungsgemäßen Ausführungsformen mit einer Beschichtung in Form einer unterkühlten Schmelze (Zustand oberhalb von Tg) von solchen mit einer Beschichtung in Form einer glasartigen festen Lösung unterschieden werden können.

Bei den erfindungsgemäßen Beschichtungen in Form einer unterkühlten Schmelze liegt die Glasübergangstemperatur Tg vorzugsweise unterhalb von 30°C, weiter bevorzugt unterhalb von 20°C, noch weiter bevorzugt unterhalb von 10°C und insbesondere bevorzugt untzerhalb von 0°C. Zudem sei angemerkt, dass es nicht entscheidend ist, ob bei der Lagerug der erfindungsgemäß beschichteten medizinischen Vorrichtung oder dem erfindungsgemäß beschichteten Katheterballon oder dem erfindungsgemäß beschichteten Stent die Glasübergangstemperatur Tg unterschritten wird. Wichtig ist, dass bei der Einführung oder Implantation der erfindungsgemäß beschichteten medizinischen Vorrichtung oder dem erfindungsgemäß beschichteten Katheterballon oder dem erfindungsgemäß beschichteten Stent die Temperatur oberhalb von Tg liegt. Da die Körpertemperatur gewöhnlich 37°C beträgt, muss Tg daher unter 37°C, vorzugsweise unterhalb 30°C, weiter bevorzugt unterhalb 20°C, noch weiter bevorzugt unterhalb 10°C und insbesondere bevorzugt unterhalb 0°C liegen.

Überraschenderweise hat sich gezeigt, dass streichfeste Lösungen für die Beschichtung einer medizinischen Vorrichtung enthaltend mindestens einen Wirkstoff besonders geeignet sind um eine erfolgreiche lokale Applikation des Wirkstoffs und besonders von hydrophoben Wirkstoffen zu vermitteln. Lösungen haben aufgrund ihrer Struktur (geringer Ordnungsgrad) den Vorteil, dass in ihnen keine starken Gitterkräfte auftreten, die einer schnellen Auflösung des Trägers und einer Freisetzung des Wirkstoffs entgegenstehen. Streichfeste Lösungen, deren Fließfähigkeit stark herabgesetzt ist bzw. deren Viskosität stark erhöht ist, haben zusätzlich die Fähigkeit während der Einführung durch teilweise mehr als einen Meter Blutgefäßstrecke auf der medizinischen Vorrichtung zu verbleiben. Es hat sich gezeigt, dass dies auch während des Prozesses der Implantierung oder der Einführung mittels Katheter der Fall ist. Die streichfeste Lösung, wie hierin definiert, kann auch als eine Lösung mit einer gummiartigen, gelartigen, zähflüssigen, halbfesten, dickviskosen oder hochviskosen Konsistenz beschrieben werden. Das heißt, die streichfesten Beschichtungen weisen in Ruhe einen festkörperartigen Zustand auf, beginnen sich aber unter der Einwirkung äußerer Kräfte zu verformen und zu fließen. Der bei der Dilatation aufgebaut Druck kann als eine solche äußere Kraft angesehen werden, welche die Beschichtung in Form einer streichfesten Lösung verformt.

In den erfindungsgemäß verwendeten, streichfesten Lösungen liegt der Wirkstoff molekulardispers verteilt in einem Träger vor, so dass er bei Kontakt mit dem Lösungsmedium (wie Blut oder andere Körperflüssigkeiten) bereits gelöst ist. Im Idealfall wird bei einer streichfesten Lösung die Geschwindigkeit der Wirkstofffreisetzung allein von der Lösungsgeschwindigkeit des Trägers bestimmt. Der Wirkstoff wird aufgrund des Wegfalls der Schmelzenthalpie beim Lösungsvorgang schneller freigesetzt als alle seine kristallinen oder kolloidalen Formen.

Die bei reinen amorphen Wirkstoffen häufig auftretende Rekristallisation während einer Lagerung kann durch streichfeste Lösungen verhindert werden. Durch die Lösung des Wirkstoffs im Träger liegen dessen Moleküle vornehmlich getrennt vor, so dass die Rekristallisation dadurch erschwert ist.

Bevorzugt sind medizinische Vorrichtungen, wobei das Mengenverhältnis des mindestens einen Wirkstoffs und der mindestens einen Trägersubstanz von 90 Gew.-% Wirkstoff zu 10 Gew.-% der Trägersubstanz bis 10 Gew.-% Wirkstoff zu 90 Gew.-% der Trägersubstanz beträgt.

Bevorzugt werden in dieser Anmeldung alle antirestenotisch wirksamen Substanzen und Wirkstoffe zur Behandlung und Vorbeugung von Erkrankungen des Bluts und der blutbildenden Organe, insbesondere antithrombotische und gerinnungshemmende Wirkstoffe. Bevorzugt sind zudem antiproliferative, antimikrotubuli, antimitotische und cytostatische Wirkstoffe.

Bevorzugt sind medizinische Vorrichtungen, bei denen der mindestens eine Wirkstoff ausgewählt wird aus der Gruppe umfassend oder bestehend aus:
Taxanen wie z.B. Paclitaxel, Docetaxel, die Limus-Verbindungen wie z.B. Rapamycin (Sirolimus), Biolimus A9, Zotarolimus, Everolimus, Myolimus, Novolimus, Pimecrolimus, Tacrolimus, Ridaforolimus, Temsirolimus, Lapachon, Vitamin K, Vitamin D, Propofol, Diazepam. Besonders bevorzugt sind Taxane und Rapamycin und dessen Derivate (Limus-Verbindungen), Cumarin, Cumarin-Derivate, Heparin, Heparin-Derivate, Dabigatran, Fondaparinux, Hirudin, Lepirudin, Rivaroxaban und Calcium-Komplexbildner.

Dabei sind die folgenden Wirklstoffe besonders bevorzugt:Taxane wie z.B. Paclitaxel, Docetaxel als auch die Limus-Verbindungen wie z.B. Rapamycin (Sirolimus), Biolimus A9, Zotarolimus, Everolimus, Myolimus, Novolimus, Pimecrolimus, Tacrolimus, Ridaforolimus und Temsirolimus. Es ist auch bevorzugt, wenn der Wirkstoff Paclitaxel in Kombination mit einem der anderen hierin genannten Wirkstoffe aufgebracht wird.

Geeignete Träger sind Substanzen, in denen der mindestens eine Wirkstoff löslich ist. Geeignete Träger sind zudem Verbindungen, die durch eine große Anzahl auftretender Wasserstoffbrückenbindungen die Kristallisation in einer unterkühlten Schmelze oder in einer Lösung verhindern bzw. erschweren und gleichzeitig eine Erwärmung bis zur Schmelztemperatur ohne Zersetzung ermöglichen. Eine wesentliche Voraussetzung für eine geeignete Trägersubstanz, ist die Mischbarkeit von Wirkstoff und Träger im geschmolzenen Zustand, sofern eine streichfeste Lösung im Schmelzverfahren hergestellt werden soll. Oder der Wirkstoff und die Trägersubstanz müssen beide in dem gleichen Lösungsmittel, bzw. Lösungsmittelgemisch löslich sein, sofern eine streichfeste Lösung im Lösungsverfahren hergestellt werden soll. Eine geeignete reine Trägersubstanz kann bei Raumtemperatur sowohl fest als auch flüssig sein. Es gehört zum Standardwissen eines jeden Chemielaboranten zu bestimmen, welche Wirkstoffe sich in welchen Trägersubstanzen und/oder Lösungsmitteln lösen. Sind Hilfsstoffe anwesend, so sollten oder müssen sich auch diese im Trägerstoff oder Lösungsmittel lösen. Geeignete Systeme aus Wirkstoff, Trägerstoff und optional Hilfsstoff und/oder optional Lösungsmittel oder Lösungsmittelgemisch zu finden erfolgt mit einfachen Standardlöslichkeitsversuchen und gehört zum Basiswissen eines Fachmanns und sogar eines jeden Laboranten.

Bevorzugt sind medizinische Vorrichtungen, bei denen die mindestens eine Trägersubstanz ausgewählt wird aus der Gruppe umfassend oder bestehend aus Polyether, Polylactonsäure, Polyethylenglykol (PEG), Poly(N-vinyl)-Pyrrolidon, N-Dodecyl-pyrrolidon, N-Decyl-pyrrolidon, N-Octyl-pyrrolidon, Polyvinylalkohole, Derivate von Polyvinylalkoholen, glycolierte Polyester, Polyphosphoester, Polyethylenoxidpropylenoxid, Polyethylenoxid, Hyaluronsäure, Copolymere mit PEG und Polypropylenglycol, Lipide, Phospholipide, Polyacrylsäure, Polyacrylate, Carboxymethylchitosane, Vanillin, Farnesol, Sorbit, Gelatine, Derivate von Gelatine, Fettsäurepartialglyceride mit einem Monoanteil von 50 bis 95 Gew.-%, Cellulose, Derivat von Cellulose, Hydroxypropylcellulose, Ethylcellulose, Stärke, Derivate von Stärke, Dextrine, Dextrane, α-Cyclodextrin, β-Cyclodextrin, γ-Cyclodextrin, Dimethyl-β-cyclodextrin, 2-Hydroxypropyl-β-cyclodextrin, Sterine, Cholesterol, Gallensäuren, Cholsäure, Lithocholsäure, N-Alkyllactame, N-Dodecylcaprolactam, N-Decyl-caprolactam, N-Octylcaprolactam, N-Dodecylvalerolactam, N-Decyl-valerolactam, N-Octylvalerolactam, Polyoxyethylen-Sorbitan-Monolaurate, Polyoxyethylen-Sorbitan-Monopalmitate, Polyoxyethylen-Sorbitan-Monostearat, Sorbitan-Monolaurat, Sorbitan-Monopalmitat, Sorbitan-Monostearat, Polyoxyethylen-oleylether, Polyoxyethylenoleylether, Bis-[alpha-methyl-(4-methyl-benzyl)]-phenyl-polyglykolether, Bis-[alpha-methyl-(4-n-dodecyl)]-phenyl-polyglykolether, Bis-(4-methyl-benzyl)-phenyl-polyglykolether, Bis-(4-n-dodecyl-benzyl)-phenyl-polyglykolether, Tris-[alpha-methyl-(4-methyl-benzyl)]-phenyl-polyglykolether, Nonylphenol-polyglykolether und Nonylphenol-diglykolether.

Bevorzugt sind Vorrichtungen, bei denen die mindestens eine Trägersubstanz ausgewählt wird aus der Gruppe umfassend oder bestehend aus: Poly(N-vinyl)-Pyrrolidon, Derivaten von Polyvinylalkoholen, glycolierte Polyester, Polyacrylsäure, Polyacrylate, Sorbit, Gelatine, Stärke, Derivate von Stärke, Cholsäure, Lithocholsäure und Polycarbonaturethane.

Insbesondere bevorzugt sind medizinische Vorrichtungen, bei denen die mindestens eine Trägersubstanz ausgewählt wird aus der Gruppe umfassend oder bestehend aus Dexpanthenol, Hyaluronsäure, Vanillin, Carboxymethylchitosan oder Polyethylenglykol.

Nicht bevorzugt oder nicht erfindungsgemäß eingesetzt werden Öle, Fette oder Wachse zur Herstellung einer erfindungsgemäßen Beschichtung in Form einer streichfesten Lösung. Werden Polymere für die Herstellung der erfindungsgemäßen Beschichtung eingesetzt, dann sind niedermolekulare Polymere bevorzugt, welche einen Polymerisationsgrad n (= Anzahl der Monomereinheiten) vorzugsweise unterhalb von 100 (n < 100), weiter bevorzugt unterhalb von 80 (n < 80), noch weiter bevorzugt unterhalb von 60 (n < 60), noch weiter bevorzugt unterhalb von 50 (n < 50), noch weiter bevorzugt unterhalb von 40 (n < 40), noch weiter bevorzugt unterhalb von 30 (n < 30) und insbesondere bevorzugt unterhalb von 20 (n < 20) haben. Wird Polyethylenglykol verwendet so ist eine mittlere Molekülmasse von 1.000 bis 10.000 g/mol bevorzugt. Dieser Absatz gilt für alle hierin offenbarten Ausführungsformen und nicht nur für die vorgenannte.

Eine weitere Ausführungsform ist die Verwendung eines Kristallisationsinhibitors als Hilfsstoff in der Beschichtung der erfindungsgemäßen medizinischen Vorrichtungen. Ein Kristallisationsinhibitor verhindert aufgrund spezifischer Interaktionen mit Wirkstoffmolekülen die ordnungsgemäße Zuammenlagerung der Arzneistoffmoleküle zu einem Subnukleus und die Kristallisation bzw. Rekristallisation.

Zudem kann die Viskosität der Beschichtung durch den Zusatz eines Hilfsstoffs erhöht werden, wenn durch die Verteilung des Hilfsstoffs im Träger die molekulare Beweglichkeit des Wirkstoffs im System herabgesetzt wird. Die vorliegende Erfindung betrifft daher auch medizinische Vorrichtungen, wobei deren Oberfläche vollständig oder teilweise mit einer streichfesten Lösung enthaltend mindestens einen molekulardispers verteilten Wirkstoff, mindestens eine Trägersubstanz und mindestens einen Hilfsstoff beschichtet ist. Es ist bevorzugt, wenn Anteile von maximal 15 Gew.-%, noch weiter bevorzugt maximal 10 Gew.-%, noch weiter bevorzugt maximal 5 Gew.-% und ganz besonders bevorzugt maximal 1 Gew.-% des Hilfsstoffs in der erfindungsgemäßen Beschichtung enthalten sind. Die hier gemachten Angaben zu den Gew.-% des Hilfsstoffs beziehen sich auf das Gesamtgewicht der Zusammensetzung, die als Beschichtung der erfindungsgemäßen Vorrichtungen dient oder anders gesagt auf das Gesamtgewicht der erfindungsgemäßen Beschichtung. Die Hilfsstoffe sind dabei vorzugsweise in der Lage die molekulare Beweglichkeit der Wirkstoffmoleküle in der streichfesten Lösung wesentlich zu erniedrigen oder verhindern durch die Wechselwirkung mit dem Wirkstoff die ordnungsgemäße Zusammenlagerung der Wirkstoffmoleküle zu einem Kristall. Sind in der Beschichtung noch Rückstände von Lösungsmitteln enthalten, so betragen die Reste an Lösungsmitteln weniger als 5 Gew.-% bezogen auf das Gesamtgewicht der erfindungsgemäßen Beschichtung.

Die Erfindung bezieht sich unter anderem auf medizinische Vorrichtungen insbesondere Stents sowie Katheterballons mit oder ohne Stent, wobei die Oberfläche der Vorrichtung vollständig oder teilweise oder zumindest teilweise mit einer streichfesten Lösung enthaltend mindestens einen Wirkstoff und eine Trägersubstanz beschichtet ist und die streichfeste Lösung auf der Oberfläche der Vorrichtung des Weiteren optional einen Hilfsstoff enthalten kann.

Die Erfindung bezieht sich unter anderem auf medizinische Vorrichtungen, wobei der Hilfsstoff bevorzugt ein Kristallisationsinhibitor ist und ausgewählt wird aus der Gruppe umfassend oder bestehend aus:
Zuckeralkohole wie beispielsweise : Glucose, Mannitol, Isomalt (Palatinitol), Lactitiol und Maltitol, Phenole und Biphenole, Harnstoff, Ölsäure, Fettsäuren, Lecithin, Sojalecithin, Alkylglycoside, Glycerin, Poly(N-vinyl)-Pyrrolidon, N-Dodecyl-pyrrolidon, N-Decyl-pyrrolidon, N-Octyl-pyrrolidon, Sorbitantristearat, Saccharoseester und Polyglycerinester von Fettsäuren, Carbamidsäureester, hochdisperses Siliciumdioxid, Röntgen kontrastmittel.

Weitere bevorzugte Hilfsstoffe sind Verdickungsmittel, Geliermittel oder Bindemittel. Die Verdickungsmittel dienen im Sinne der Erfindung zur Herabsetzung der molekularen Beweglichkeit. Es gibt auch höher molekulare Kristallisationsinhibitoren, die auch als Hilfsstoffe zur Erhöhung der Viskosität eingestuft werden können. Daher wird im Folgenden nur allgemein von Hilfsmitteln gesprochen. Verdickungsmittel können in der Lage sein Wasser zu binden. Dadurch kommt es zu einer Erhöhung der Viskosität, bzw. können sie dazu dienen, die hohe Viskosität insbesondere auch während der Lagerung zu erhalten, indem sie z.B. Kondenswasser binden.

Bei den meisten Verdickungsmitteln handelt es sich um lineare oder verzweigte Makromoleküle (z. B. Polysaccharide oder Proteine), die durch intermolekulare Wechselwirkungen, wie Wasserstoffbrücken, hydrophobe Wechselwirkungen oder Ionenbeziehungen miteinander interagieren können. Vorzugsweise sind die Verdickungsmittel Pflanzengummis. Pflanzengummis sind Polysaccharide natürlicher Herkunft, die dazu in der Lage sind, die Viskosität einer Lösung selbst bei geringer Konzentration stark zu erhöhen. In der Nahrungsmittelindustrie werden sie als Verdickungsmittel, Geliermittel und Stabilisatoren verwendet.

Die Erfindung bezieht sich daher unter anderem auf medizinische Vorrichtungen, wobei der Hilfsstoff bevorzugt ein Verdickungsmittel ist und ausgewählt wird aus der Gruppe umfassend oder bestehend aus: Agar, Alginsäure, Alginat, Chicle, Dammar, Eibisch-Extrakt, Gellan (E 418), Guarkernmehl (E 412), Gummi arabicum (E 414), Gummi aus Wegerichkernspelze, Gummi aus Fichtensaft, Johannisbrotkernmehl (E 410), Karaya (E 416), Konjakmehl (E 425), Mastix, Pectin, Tarakernmehl (E 417), Traganth (E 413), Xanthan (E 415), Carrageenan, Cellulose, Celluloseether, Gelatine, Sago und Stärke.

Voraussetzung für einen Hilfsstoff ist, dass sich der entsprechende Hilfsstoff in der Trägersubstanz löst, um mit den gelösten Wirkstoffmolekülen auf molekularer Ebene wechselwirken zu können.

Die streichfeste Lösung kann durch Schmelzeinbettung, Kopräzipitation, Verdunsten eines Lösemittels oder Eindicken einer Lösung oder einer Kombination dieser Methoden hergestellt werden. Die Herstellung streichfester Lösungen im Schmelzverfahren besteht aus einer Mischung von Träger, Wirkstoff und eventuell Hilfsstoffen im geschmolzenen Zustand. Diese Mischung hat bevorzugt entweder einen Schmelzpunkt unterhalb der Körpertemperatur und auch unterhalb der Lagerungstemperatur oder verbleibt im Bereich einer unterkühlten Schmelze.

Zur Beschichtung wird bevorzugt eine Lösung eines Wirkstoffs und einer Trägersubstanz eventuell in Kombination mit einem Hilfsstoff in einem leichtflüchtigen Lösungsmittel aufgebracht. Beim Verdunsten des Lösungmittels bleibt eine extrem hochviskose, homogene Lösung aus der Trägersubstanz, eventuell einem Hilfsstoff und dem Wirkstoff übrig, die den Wirkstoff in molekulardisperser Form enthält und keine Kristalle oder Partikel des Wirkstoffs aufweist. Die Mischung aus Trägersubstanz und Wirkstoff bilden dabei eine streichfeste Lösung.

Da eine Kristallisation des Wirkstoff verhindert werden soll, sind Lösungsmittel mit hohem Dampfdruck bevorzugt, dass heißt Lösungsmittel, die schon bei niedrigen Temperaturen (z. B. 50°C) als Gas vorliegen oder bei Raumtemperatur leicht flüchtig sind. Der Siedebereich der Lösungsmittel liegt vorzugsweise zwischen 0 und 150 C und noch weiter bevorzugt zwischen 10°C und 100°C und besonders bevorzugt zwischen 20°C und 50°C. Als organische Lösungsmittel eignen sich Acetonitril, Dimethylsulfoxid, Ether wie beispielsweise Dioxan, Tetrahydrofuran (THF), Petrolether, Diethylether, Methyl-tert.-butylether (MTDC), Ketone wie beispielsweise Aceton, Butanon oder Pentanon, Alkohole wie beispielsweise Methanol, Ethanol, Propanol, Isopropanol, Carbonsäuren wie beispielsweise Ameisensäure, Essigsäure, Propionsäure, Amide wie beispielsweise Dimethylformamid (DMF) oder Dimethylacetamid, aromatische Lösungsmittel wie beispielsweise Toluol, Benzol, Xylol, reine Kohlenwasserstofflösungsmittel wie beispielsweise Pentan, Hexan, Cyclohexan, halogenierte Lösungsmittel wie beispielsweise Chloroform, Methylenchlorid, Tetrachlorkohlenstoff, sowie Carbonsäureester wie beispielsweise Essigsäureethylester.

Als Verfahren zur schnellen Unterkühlung einer Schmelze sind die Schmelzerstarrung, die Sprüherstarrung und das Hot-Spin-Melting geeignet. Eine generelle Vorraussetzung für die Herstellung streichfester Lösungen im Schmelzverfahren ist die Mischbarkeit von Träger, Wirkstoff und Hilfsstoffen im geschmolzenen Zustand. Nur durch Lösung des Wirkstoffs im geschmolzenen Träger lässt sich die molekulardisperse Verteilung erreichen, die zur Bildung einer streichfesten Lösung im Sinne der Erfindung notwendig ist. Als unmischbar werden hier Wirkstoffe bezeichnet, die in einer Konzentration von ≥1% mit der Schmelze eines Trägers nicht mischbar sind. Die Durchführung derartiger Schmelzversuche liegt im Durchschnittskönnen eines Fachmanns und sogar eines jeden Chemielaboranten.

Es wurde gefunden, dass ein Beschichtungsverfahren der folgenden Art die gestellte Aufgabe besonders gut löst. Dieses Verfahren zur Beschichtung eines Katheterballons umfasst die folgenden Schritte:
a) Bereitstellung einer unbeschichteten medizinischen Vorrichtung, und
b) Bereitstellen einer Beschichtungslösung enthaltend mindestens einen antirestenotischen Wirkstoff und eine Trägersubstanz in mindestens einem Lösungsmittel und
c) Aufbringen der Beschichtungslösung aus dem mindestens einen Wirkstoff und Trägersubstanz mittels Sprühen, Tauchen, Streichen, Pinseln, Pipettieren, Gasphasenabscheidung oder Spritzen und
d) Verdunsten oder Trocknen des Lösungsmittels bei erhöhter Temperatur und/oder unter vermindertem Druck.

Die Erfindung umfasst auch beschichtete medizinische Vorrichtungen erhältlich nach obigem Verfahren.

Die Oberfläche kann zusätzlich mit einer hämokompatiblen Schicht als Basisbeschichtung versehen werden, die durch kovalente Immobilisierung von semisynthetischen Heparinderivaten wie z.B. desulfatiertem reacetyliertem Heparin oder Chitosanderivaten wie z.B. N-carboxymethyliertes, partiell N-acetyliertes Chitosan aufgebracht wird.

Nachdem die erfindungsgemäßen medizinischen Vorrichtungen in den Körper eingebracht oder implantiert wurden, wird die Beschichtung gegen das umliegende Gewebe gepresst, bleibt dort wegen der hohen Viskosität haften und gibt den Wirkstoff in molekulardisperser, gelöster und/oder in mikrokristalliner Form an das Gewebe ab. Im letzten Fall wird die Bildung von Mikrokristallen durch Kontakt mit dem wässrigen System Blut oder dem wässrigen System extrazelluläre Flüssigkeit ausgelöst, weil erfindungsgemäß keine Kristalle in der Beschichtung vorhanden sind.

Im Folgenden werden Methoden zur Charakterisierung streichfester Lösungen offenbart. Dabei wird insbesondere die Abgrenzung zu Suspensionen, wie Pasten, beschrieben.

Bereits eine makroskopische Untersuchung ermöglicht eine Aussage über das Vorliegen einer Lösung oder einer Suspensionen. Suspensionen erscheinen milchig trübe, da es ähnlich einer Emulsion zum Einschluss von Tröpfchen der niedriger konzentrierten Phase im Träger kommt. Ein kristalliner Wirkstoff in einem Träger, weist makroskopisch auftretende Trübungen des transparenten Trägers auf. Eine Ausnahme besteht nur, wenn sich die Brechungsindices der Wirkstoffphase und der Phase des Trägers nicht unterscheiden. Eine auftretende Trübung oder ein milchiges Aussehen ist daher entweder ein Anzeichen für eine (Re)Kristallisation des Wirkstoffs oder für eine Suspension.

Streichfeste Lösungen sehen also bevorzugt klar oder transparent aus. Dies schließt nicht aus, dass sie farbig sind, sowie auch glasfarbig aber dennoch klar bzw. transparent sind. Es kann zur besseren Qualitätskontrolle der Beschichtung vorteilhaft sein, geringe Mengen eines Farbstoffs in die Beschichtung einzubringen.

Durch thermische Analyseverfahren können temperaturabhängige Veränderungen in einer Probe beobachtet werden. Dies sind insbesondere Phasenübergänge erster und zweiter Ordnung. Phasenübergänge erster Ordnung sind Phasenübergänge, die mit einer Aufnahme oder Abgabe von latenter Wärme verbunden sind. Die Auftragung der Enthalpie H über der Temperatur zeigt dann bei der Umwandlungstemperatur eine Stufe. Phasenübergänge zweiter Ordnung verlaufen ohne Aufnahme oder Abgabe von latenter Wärme. Die Enthalpie zeigt hier bei der Umwandlungstemperatur einen Knick, die Wärmekapazität eine Stufe in der Auftragung über der Temperatur.

Eine Unterscheidung zwischen einer festen Lösung und einer unterkühlten Schmelze, welche noch erfindungsgemäß wäre oder einer hochviskosen Lösung kann mit Hilfe der Dynamischen Differenzkalorimetrie (DDK) (engl: Differential Scanning Calorimetry (DSC)) getroffen werden. Mittels DDK kann auch eine Aussage über die Mischbarkeit von Stoffen getroffen werden. Bei einer heterogenen Mischung können die Phasenübergänge beider Reinsubstanzen beobachtet werden, während bei homogenen Mischungen nur noch die Phasenübergänge der Mischung beobachtet werden kann.

Bei der Wärmestrom-DDK (englisch: heat flux DSC), befinden sich Probe und Referenz auf einer wärmeleitenden Scheibe (Disk type measuring system), welche eine gute Wärmeleitfähigkeit besitzt und unter welcher die Temperaturfühler sitzen. Gemessen wird die Differenz der Wärmeströme zwischen Probe und Referenz. Sind Probe und Referenz gleich ist die Wärmestromdifferenz null. Verändert sich während der Messung eine Probe, z. B. durch Phasenumwandlung, Schmelzen, Verdampfen, entsteht eine Differenz im Wärmestrom.

Schmelzvorgänge sind dabei durch einen positiven Wärmestrom und Kristallisationsvorgänge durch einen negativen Wärmestrom gekennzeichnet. Der Übergang einer amorphen Substanz vom glasartigen Zustand in den Bereich der unterkühlten Schmelze ist durch eine plötzliche Zunahme der Wärmekapazität gekennzeichnet, ohne dass dabei ein zusätzlicher Wärmetransfer erfolgt. Während einer DDK kann man daher einen erhöhten differentiellen Wärmestrom zur Probe nach Überschreiten der Glasübergangstemperatur Tg messen.

Mit Hilfe von Freisetzungsuntersuchungen kann man ebenfalls eine Charakterisierung der Beschichtungen vornehmen, das heißt die Art der Verteilung eines Wirkstoffs im Träger kann bestimmt werden.

Der Beschichtung der medizinischen Vorrichtung wird dabei die Möglichkeit gegeben, sich in einem Freisetzungsmedium (angepasst der entsprechenden, das Implantat umgebenden Flüssigkeit, wie z.B. eine physiologische Kochsalzlösung, Blut) zu lösen, bzw. den Wirkstoff freizusetzen. Der in der Trägersubstanz gelöste Wirkstoff besitzt aufgrund des Wegfalls der Schmelzenthalpie beim Lösungsvorgang bzw. Mischungsvorgang einer streichfesten Lösung eine höhere thermodynamische Löslichkeit. Damit erhöhen sich Lösungsgeschwindigkeit und Löslichkeit maximal. Insbesondere bei schneller Auflösung des Trägers kann es zur vorübergehenden Übersättigung an Wirkstoff im Freisetzungsmedium kommen. Aus dem Ausmaß der Übersättigung und der Geschwindigkeit der Wirkstofffreisetzung im Freisetzungsmedium lassen sich zudem Rückschlüsse auf die Bioverfügbarkeit ziehen.

Die vorliegende Erfindung betrifft vorzugsweise einen Katheterballon mit oder ohne Stent, wobei die Oberfläche des Katheterballons als auch die Oberfläche des Stents vollständig oder teilweise mit einer streichfesten Lösung enthaltend mindestens einen Wirkstoff beschichtet ist. Es ist bevorzugt, wenn es sich dabei um einen antirestenotischen wasserunlöslichen bzw. schwerlöslichen Wirkstoff handelt. Die bereits in ihrer antirestenotischen Wirksamkeit auf Stents erwiesenen Taxane wie z.B. Paclitaxel, Docetaxel als auch die Limus-Verbindungen wie z.B. Rapamycin (Sirolimus), Biolimus A9, Zotarolimus, Everolimus, Myolimus, Novolimus, Pimecrolimus, Tacrolimus, Ridaforolimus, Temsirolimus werden hierbei bevorzugt eingesetzt. Die Wirkstoffwahl beschränkt sich aber nicht nur auf diese beiden Gruppen. Es können grundsätzlich beliebige Wirkstoffe eingesetzt werden, welche der Entstehung einer Restenose entgegenwirken und beipsielswise antiproliferativ, antiangiogen oder antimigrativ sind.

Als Katheterballons können die herkömmlichen Katheterballons, Bifurkationsballons und auch Faltenballons oder Spezialballons wie beispielsweise der Schlitzballon oder der Scoringballon eingesetzt werden.

Zur Beschichtung wird bevorzugt eine Lösung eines Wirkstoffs und einer Trägersubstanz eventuell in Kombination mit einem Hilfsstoff in einem leichtflüchtigen Lösungsmittel aufgebracht. Beim Verdunsten des Lösungsmittels bleibt eine extrem hochviskose homogene Lösung aus der Trägersubstanz, eventuell einem Hilfsstoff und dem Wirkstoff übrig, die den Wirkstoff in molekulardisperser Form enthält und keine Kristalle oder Partikel des Wirkstoffs aufweist. Die Trägersubstanz und der mindestens eine Wirkstoff bilden dabei eine streichfeste Lösung.

Bei der Ballondilatation wird die Beschichtung gegen die Gefässwand gepresst, bleibt dort wegen der hohen Viskosität haften und gibt den Wirkstoff in molekulardisperser, gelöster und/oder in mikrokristalliner Form an die Gefässwand ab. Im letzten Fall wird die Bildung von Mikrokristallen durch Kontakt mit dem wässrigen System Blut ausgelöst nachdem die Beschichtung in Form einer streichfesten Lösung auf die Gefäßwand übertragen wurde, weil die erfindungsgemäße Beschichtung keine Wirkstoffkristalle oder Wirkstoffpartikel aufweist.

Es ist bevorzugt, wenn die gesamte Ballonoberfläche gleichmäßig mit einer streichfesten Lösung enthaltend den Wirkstoff beschichtet ist, also auch der Bereich unter den Falten sowie die nicht durch die Falten abgedeckte Oberfläche des Katheterballons. Ferner ist bevorzugt, wenn eine gleichmäßige Verteilung des mindestens einen Wirkstoffes auf der Ballonoberfläche vorliegt. Im Falle von Valvuloplastieballons ist eine Beschichtung im mittleren Drittel des Ballons bevorzugt, weil nur dieser Bereich des Ballons mit der Herzklappe in Berührung kommt. Das distale und ventrale Ende des Katheterballons ist dann vorzugsweise unbeschichtet, um Wirkstoff einzusparen und die Wirkstoffexposition des Patienten zu minimieren.

Vorzugsweise werden die erfindungsgemäß beschichteten Katheterballons ohne aufgesetzten Stent verwendet, jedoch ist eine Verwendung mit einem biostabilen oder bioabbaubaren Stent ebenfalls möglich. Dieser Stent kann unbeschichtet sein, zusammen mit dem Katheterballon erfindungsgemäß beschichtet werden oder bereits vor dem Aufsetzen, d.h. Crimpen auf den Katheterballon beschichtet sein mit demselben oder mit einem anderen Wirkstoff als auch mit derselben oder einer anderen Trägersubstanz.

Dabei können sowohl die gesamte Oberfläche des Katheterballons oder nur die Falten des Ballons als auch die Oberfläche des Stents vollständig oder teilweise mit einer streichfesten Lösung enthaltend mindestens einen antirestenotischen Wirkstoff und eine Trägersubstanz beschichtet sein. Der Stent kann natürlich auch mit einer anderen Beschichtung der Wahl versehen sein. Auch die Freisetzungskinetik des verwendeten antirestenotischen Wirkstoffes auf dem Stent kann sich von der Freisetzungskinetik des verwendeten Wirkstoffs auf dem Katheterballon unterscheiden. Somit stellt das erfindungsgemäße expandierbare System auch eine Kombination aus Katheterballon mit Stent bereit. Ferner kann der Stent nur mit einer hemokompatiblen Beschichtung mit oder ohne einen Wirkstoff versehen sein. Katheterballon mit gekrimptem Stent können gleichzeitig beschichtet werden, oder Katheterballon und Stent werden separat beschichtet und danach wird der beschichtete Stent auf den beschichteten Katheterballon gekrimpt.

Der Begriff "Beschichtung" soll nicht nur eine Beschichtung der Oberfläche des Katheterballons sondern auch eine Befüllung oder Beschichtung von Falten, Kavitäten, Poren, Mikronadeln oder anderen befüllbaren Räumen auf oder zwischen oder in dem Ballonmaterial umfassen.

Die erfindungsgemäße Beschichtung zeichnet sich dadurch aus, dass keine Feststoffe oder feste Partikel darin enthalten sind. Somit kann es notwendig sein, dem Wirkstoff so viel Kristallisationsinhibitor zuzusetzen, dass nach Verdunstung oder Entfernung des Lösungsmittels der Beschichtungslösung der Wirkstoff nicht kristallisiert oder sich feste Wirkstoffpartikel in dem Träger bilden.

Der Begriff "Kristallisationsinhibitor wie hierin benutzt, beschreibt Substanzen, die bei Zugabe zu einem Wirkstoff in einer streichfesten Lösung die Kristallbildung verhindern oder vermindern.

Als "hydrophil" werden Trägersubstanzen (T_{OW}) bezeichnet mit einem Verteilungskoeffizienten zwischen n-Oktanol und Wasser von T_{OW} < 6.30 (log T_{OW} < 0.80), bevorzugt T_{OW} < 1.80 (log T_{OW} < 0.26), weiter bevorzugt T_{OW} < 0.63 (log T_{OW} < -0.20) und noch weiter bevorzugt T_{OW} < 0.40 (log T_{OW} < -0.40).

Als "hydrophob" oder "lipophil" werden antirestenotische Wirkstoffe bezeichnet mit einem Verteilungskoeffizienten zwischen n-Butanol und Wasser von ≥0,5, vorzugsweise ≥0,7 weiter bevorzugt ≥0,9 und insbesondere bevorzugt ≥1,1.

Überraschenderweise hat sich gezeigt, dass streichfeste Lösungen für eine Beschichtung enthaltend Wirkstoffe und bevorzugt hydrophobe antirestenotische Wirkstoffe besonders geeignet sind und folgende Eigenschaften zur erfolgreichen lokalen Applikation vermitteln:
1) die Kontaktzeit des Katheterballons reicht zur Übertragung einer geeigneten therapeutischen Wirkstoffmenge in und an die Gefässwand aus,
2) während des Kontaktes bei der Ballondilatation bleibt genügend wirkstoffenthaltendes Beschichtungsmaterial an der Gefässwand haften, um den gewünschten therapeutischen Effekt zu gewährleisten
   und,
3) die auf den Katheterballons vorhandene, wirkstoffenthaltende Beschichtung weist eine höhere Affinität zur Gefässwand als zur Ballonoberfläche auf, so dass eine optimale Übertragung des Wirkstoffes auf den Zielort erfolgen kann.
4) Die Beschichtung ist nicht spröde, sondern sehr zähflüssig und reißt daher während der Dilatation nicht auf, so dass sich keine Bruchstücke bilden können, welche in den Blutkreislauf gelangen.
5) Die hohe Viskosität der Beschichtung führt jedoch dazu, dass die Beschichtung trotzdem stabil genug ist, um während des Einführens des Katheters bis zum Applikationsort am Katehterballon sicher zu haften.
6) Es werden keine Partikel bei der Dilatation freigesetzt.
7) Es werden keine Wirkstoffpartikel bei der Dilatation freigesetzt und auf die Gefäßwand übertragen.
8) Der Wirkstoff wird in molekularer Form, d.h. als einzelne Moleküle auf den zu behandelnden Gefäßabschnitt übertragen und nicht als Kolloide, Kristalle oder amorphe Partikel, wodurch die höchste Wirksamkeit erzielt wird und die eingesetzte Wirkstoffmenge minimiert werden kann.
9) Die hohe Viskosität der Beschichtung und grössere Affinität der Beschichtung zur Gefässwand als zur Ballonoberfläche führt zu einer, im Vergleich zur reellen Kontaktzeit der Gefässwand mit dem Ballonkatheter, verlängerten Wirkstoffabgabe.
10) Die an der Gefässwand haftende Beschichtung löst sich aufgrund der hohen Viskosität ohne das Risiko der Freisetzung freier fester Partikel in den Blutkreislauf gleichmässig und ohne Rückstände zu hinterlassen im Blutstrom vollständig auf.

Daher wurde gefunden, dass ein Beschichtungsverfahren der folgenden Art die gestellte Aufgabe besonders gut löst.

Dieses Verfahren zur Beschichtung eines Katheterballons umfasst die folgenden Schritte:
a) Bereitstellen eines unbeschichteten Katheterballons mit oder ohne Stent, und
b) Bereitstellen einer Beschichtungslösung enthaltend mindestens einen antirestenotischen Wirkstoff und eine Trägersubstanz in mindestens einem Lösungsmittel
   und
c) Aufbringen der Beschichtungslösung aus mindestens einem Wirkstoff und der Trägersubstanz und mindestens einem Lösungsmittel mittels Sprühen, Tauchen, Streichen, Pinseln, Pipettieren, Gasphasenabscheidung oder Spritzen auf den unbeschichteten Katheterballon und
d) Verdunsten oder Trocknen des Lösungsmittels bei erhöhter Temperatur und/oder unter vermindertem Druck.

Bevorzugt werden dilatierbare Katheterballons mittels dieses Verfahrens beschichtet. In Schritt c) des obigen Verfahrens zur Beschichtung oder Beladung eines Katheterballons wird die Beschichtungslösung auf alle zu beladenden Oberflächen aufgebracht. Dabei kann es sich sowohl um die Oberfläche des Katheterballons oder um die Oberfläche des Katheterballons als auch die eines Stents handeln. Der Katheterballon kann auch nur teilweise beschichtet werden.

Der Katheterballon kann sowohl im gefalteten oder komprimierten Zustand beschichtet werden als auch im dilatierten oder expandierten Zustand, wobei der Ballon dann nach dem Aufbringen der Beschichtung wieder zusammengefaltet wird. Zudem kann der Katheterballon ohne Stent als auch zusammen mit einem darauf gekrimpten, d.h. aufgesetzten Stent beschichtet werden, so dass die abluminale Oberfläche des Stents als auch die Ballonoberfläche insbesondere zwischen den Stentstreben mit der streichfesten, viskösen Lösung enthaltend mindestens einen antirestenotischen Wirkstoff und mindestens eine Trägersubstanz beschichtet sind. Dabei kann auf dem Katheterballon sowohl ein unbeschichteter Stent als auch ein bereits beschichteter Stent verwendet werden. Die Stentoberfläche kann zusätzlich mit einer hämokompatiblen Schicht als Basisbeschichtung versehen sein, die durch kovalente Immobilisierung von semisynthetischen Heparinderivaten wie z.B. desulfatiertem reacetyliertem Heparin oder Chitosanderivaten wie z.B. N-carboxymethyliertes, partiell N-acetyliertes Chitosan auf die Stentoberfläche aufgebracht wurde.

Nach der Beschichtung des Katheterballons mit der Lösung aus mindestens einem antirestenotischen Wirkstoff, einem geeigneten Träger und bevorzugt einem Hilfsstoff erhält man nach Verdunsten oder Entfernen des Lösungsmittels bei erhöhter Temperatur und/oder unter vermindertem Druck eine Beschichtung bestehend aus einer streichfesten Lösung von einem antirestenotischen Wirkstoff in einem Träger, dem eventuell ein Hilfsstoff beigemischt wurde. Der antirestenotische Wirkstoff ist dabei molekulardispers in dem erstarrten bzw. hochviskosen Träger verteilt. Auf diese Weise wird ohne Veränderungen der Wirkstoffstruktur die Verwendbarkeit hydrophober Wirkstoffe auf hydrophile Systeme erweitert.

Die Beschichtung kann auch aktiv durch Erwärmung oder Anlegen von Vakuum oder im Gasstrom getrocknet werden. Bevorzugt werden Temperaturen zwischen 50°C und 100°C verwendet.

Bevorzugt werden bei der vorliegenden Erfindung in Bezug auf einen Katheterballon und oder einen Stent alle antirestenotisch wirksamen hydrophoben Substanzen wie z.B. Paclitaxel, Docetaxel, Rapamycin (Sirolimus), Biolimus A9, Zotarolimus, Everolimus, Myolimus, Novolimus, Pimecrolimus, Tacrolimus, Ridaforolimus, Temsirolimus, Cyclosporine, Vitamin K, Propofol, Diazepam. Besonders bevorzugt sind Taxane und Rapamycin und dessen Derivate (Limus-Verbindungen).

Allgemein wird der Begriff Taxan in dieser Anmeldung als Oberbegriff für Paclitaxel, Paclitaxelderivate und Paclitaxelanaloga verwendet. Der Begriff Limus-Verbindungen steht hier für Sirolimus (Rapamycin), dessen Derivate und Analoga.

Der Begriff Derivat bezeichnet einen abgeleiteten Stoff ähnlicher Struktur zu einer entsprechenden Grundsubstanz. Derivate sind Stoffe, deren Moleküle an Stelle eines H-Atoms oder einer funktionellen Gruppe ein anderes Atom oder eine andere funktionelle Gruppe besitzen bzw. bei denen ein oder mehrere Atome/funktionelle Gruppen entfernt und/oder addiert und/oder ausgetauscht wurden.

Besonders bevorzugt ist ein Katheterballon, wobei es sich bevorzugt beim mindestens einen Taxan um Paclitaxel oder dem mindestens einer Limus-Verbindung um Rapamycin handelt. Die Wirkstoffe Paclitaxel oder Rapamycin sind dabei in Kombination mit einem geeigneten Träger in der Beschichtung des Katheterballons vorhanden.

Die wirkstofffreisetzende Beschichtung auf dem Katheterballon und/oder Stent kann folgende Substanzen enthalten: Polyether, Polylactonsäure, Polyethylenglykol (PEG), Poly(N-vinyl)-Pyrrolidon, N-Dodecyl-pyrrolidon, N-Decyl-pyrrolidon, N-Octyl-pyrrolidon, Polyvinylalkohole, Derivate von Polyvinylalkoholen, glycolierte Polyester, Polyphosphoester, Polyethylenoxidpropylenoxid, Polyethylenoxid, Hyaluronsäure, Copolymere mit PEG und Polypropylenglycol, Lipide, Phospholipide, Polyacrylsäure, Polyacrylate, Carboxymethylchitosane, Lanolin, Vanillin, Sorbit, Gelatine, Derivate von Gelatine, Fettsäurepartialglyceride mit einem Monoanteil von 50 bis 95 Gew.-%, hochdisperses Siliciumdioxid, Cellulose, Derivat von Cellulose, Hydroxypropylcellulose, Ethylcellulose, Stärke, Derivate von Stärke, Dextrine, Dextrane, α-Cyclodextrin, β-Cyclodextrin, γ-Cyclodextrin, Dimethyl-β-cyclodextrin, 2-Hydroxypropyl-β-cyclodextrin, Sterine, Cholesterol, Gallensäuren, Cholsäure, Lithocholsäure, N-Alkyllactame, N-Dodecylcaprolactam, N-Decyl-caprolactam, N-Octylcaprolactam, N-Dodecylvalerolactam, N-Decyl-valerolactam, N-Octylvalerolactam, Polyoxyethylen-Sorbitan-Monolaurate, Polyoxyethylen-Sorbitan-Monopalmitate, Polyoxyethylen-Sorbitan-Monostearat, Sorbitan-Monolaurat, Sorbitan-Monopalmitat, Sorbitan-Monostearat, Polyoxyethylen-oleylether, Polyoxyethylen-oleylether, Bis-[alpha-methyl-(4-methyl-benzyl)]-phenyl-polyglykolether, Bis-[alpha-methyl-(4-n-dodecyl)]-phenyl-polyglykolether, Bis-(4-methyl-benzyl)-phenyl-polyglykolether, Bis-(4-n-dodecyl-benzyl)-phenyl-polyglykolether, Tris-[alpha-methyl-(4-methyl-benzyl)]-phenyl-polyglykolether, Nonylphenol-polyglykolether und Nonylphenol-diglykolether..

Besonders bevorzugt wird Dexpanthenol, Hyaluronsäure, Carboxymethylchitosane, Vanillin und PEG als Trägersubstanz verwendet und insbesondere Dexpanthenol.

Dexpanthenol ist unter dem Markenname Bepanthen^{®} und den Synonymen Panthothenol, D-Panthenol oder Panthenol und dem IUPAC-Namen (+)-(*R*)-2,4-Dihydroxy-*N*-(3-hydroxypropyl)-3,3-dimethylbutyramid bekannt.

Die chemische Struktur ist wie folgt:

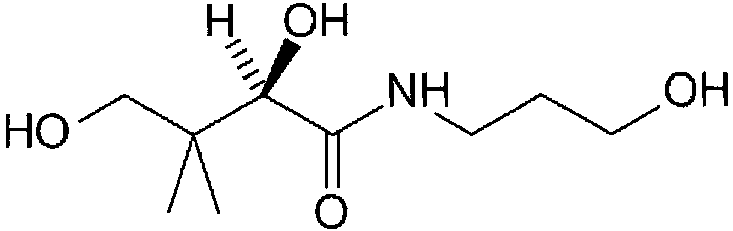

In der Regel wird eine Menge von 0,5 µg bis 50 µg Wirkstoff pro mm² Oberfläche des zu beschichtenden Ballonkatheters und bevorzugt eine Menge von 1 µg bis 20 µg Wirkstoff pro mm² Oberfläche des zu beschichtenden Ballonkatheters auf die Ballonoberfläche aufgetragen. Pro Katheterballon werden vorzugsweise 10 bis 1000 µg Wirkstoff und insbesondere bevorzugt werden pro Katheterballon 20 µg bis 400 µg auf den Ballon aufgetragen.

Als Lösungsmittel für die Mischung aus Wirkstoff und Trägersubstanz kann Chloroform, Ethanol, Methanol, Tetrahydrofuran, Aceton, Methylacetat, Essigsäureethylester, Methylenchlorid oder ihre Gemische, wie Methanol / Ethanol-Gemische, Ethanol/Wasser-Gemische verwendet werden. Ein geeignetes Lösungsmittel löst zum einen die erforderlichen Mengen an Wirkstoff und Trägersubstanz und greift das Material des Ballonkatheters nicht oder nur geringfügig an. Besonders bevorzugt werden Aceton, Ethanol und Ethylacetat.

In einem mL des gewählten Lösungsmittel werden vorzugsweise 0,1 mg - 600 mg, weiter bevorzugt 1 mg bis 500 mg und insbesondere bevorzugt 10 mg - 250 mg Wirkstoff gelöst. Ferner werden in einem mL des gewählten Lösungsmittels vorzugsweise 0,1 mg - 600 mg, weiter bevorzugt 1 mg bis 250 mg und insbesondere bevorzugt 10 mg - 250 mg Trägersubstanz gelöst.

Weiter bevorzugt ist ein Katheterballon der eine Beschichtung aufweist deren Mengenverhältnis von antirestenotischem Wirkstoff und Trägersubstanz von 90 Gew.-% antirestenotischem Wirkstoff zu 10 Gew.-% Trägersubstanz bis 10 Gew.-% antirestenotischem Wirkstoff zu 90 Gew.-% Trägersubstanz beträgt.

Ferner bevorzugt ist ein Katheterballon der eine Beschichtung aufweist deren Molverhältnis von Wirkstoff zu Trägersubstanz von 90 % Wirkstoff zu 10 % Trägersubstanz bis 10 % Wirkstoff zu 90 % Trägersubstanz beträgt. Weiter bevorzugt sind Mischungen von 1 : 5 bis 5 : 1 und noch weiter bevorzugt von 1 : 2 bis 2 : 1.

Es ist auch möglich, einen weiteren Hilfsstoff z.B. als Kristallisationsinhibitor in die Wirkstofflösung zu geben. Es eignen sich als weitere Hilfsstoffe beispielsweise biologisch verträgliche organische Stoffe, die die Beschichtungseigenschaften verbessern und die Aufnahme von Wirkstoff in das Gefäß erhöhen wie beispielsweise Aminosäuren, Vitamine, Benzoesäurebenzylester, Triethyl- und Dimethylphtalat, Fettsäureester wie Isopropylmyristat und -palmitat, Triacetin und dergleichen. Als ebenfalls gut geeignet zeigen sich Mischungen aus diesen verschiedenenen Substanzen. So zeichnet sich z.B. die Mischung aus dem Polysaccharid Carrageenan, Lecithin und Glycerin als geeignetes Wirkstoffträgersystem aus. Auch physiologisch verträgliche Salze können als Hilfsstoff zur Einlagerung des Wirkstoffs verwendet werden.

Eine weitere bevorzugte Ausführungsform der vorliegenden Erfindung umfasst einen beschichteten Katheterballon mit gekrimptem Stent.

Bei dieser Ausführungsform kann der Stent unbeschichtet sein oder die gleiche erfindungsgemäße Beschichtung aufweisen wie der Katheterballon.

Der Katheterballon mit oder ohne Stent wird entweder mit einer glasartigen festen Lösung aus antirestenotischem Wirkstoff in einem Träger mittels Sprüh-, Pinsel-, Spritzen-, Schlepp-, Roll- oder Pipettierverfahren oder Elektrospinning vollständig oder partiell beschichtet, wobei der Beschichtungslösung noch ein weiterer Hilfsstoff, wie ein Kristallisationsinhibitor, zugesetzt werden kann.

Der Katheterballon lässt sich entweder im expandierten oder im gefalteten Zustand beschichten, teilweise oder vollständig beschichten oder zusammen mit einem aufgesetzten Stent beschichten. Zudem kann die Beschichtung ausschliesslich in den Falten bzw. ausschliesslich ausserhalb der Falten erfolgen.

Die Beschichtung ist im Sprüh-, Tauch-, Pinsel-, Spritzen-, Schlepp-, Fadenschlepp-, Roll- und/oder Pipettierverfahren durchführbar. Das Pipettier-, Schlepp-, Roll- oder Spritzenverfahren eignet sich vor allem für die Anwendung auf einem gefalteten Katheterballon oder einem Faltenballon, da man mit diesem Verfahren gezielt die Wirkstofflösung oder die Mischung der aufzutragenden Substanzen in die Falten bzw. unter die Falten bringen kann. Die Trocknung des beschichteten Ballonkatheters ohne Stent oder mit Stent kann mittels Rotationstrocknung geschehen. Die Beschichtungsverfahren sind ausführlich in der WO2008086794A beschrieben.

### Pipettierverfahren - Kapillarverfahren

Dieses Verfahren ist ausführlich beschrieben in der Offenlegungsschrift der internationalen Patentanmeldung WO2008086794 S. 74 Zeile 33 bis S. 78 Zeile 9.

### Spritzverfahren oder Spritzenverfahren:

Dieses Verfahren ist ausführlich beschrieben in der Offenlegungsschrift der internationalen Patentanmeldung WO2008086794 S. 78 Zeile 12 bis S. 82 Zeile 8.

### Sprühverfahren oder Faltensprühverfahren:

Dieses Verfahren ist ausführlich beschrieben in der Offenlegungsschrift der internationalen Patentanmeldung WO2008086794 S. 82 Zeile 10 bis S. 84 Zeile 19.

### Schleppverfahren oder Tropfenschleppverfahren:

Dieses Verfahren ist ausführlich beschrieben in der Offenlegungsschrift der internationalen Patentanmeldung WO2008086794 S. 84 Zeile 21 bis S. 86 Zeile 25.

### Fadenschleppverfahren:

Dieses Verfahren ist ausführlich beschrieben in der Offenlegungsschrift der internationalen Patentanmeldung WO2008086794 S. 86 Zeile 27 bis S. 88 Zeile 3.

### Kugelschreiberverfahren oder Rollverfahren:

Dieses Verfahren ist ausführlich beschrieben in der Offenlegungsschrift der internationalen Patentanmeldung WO2008086794 S. 88 Zeile 7 bis S. 89 Zeile 6.

### Rotationstrocknung:

Dieses Verfahren ist ausführlich beschrieben in der Offenlegungsschrift der internationalen Patentanmeldung WO2008086794 S. 89 Zeile 8 bis S. 91 Zeile 8.

Unter dem Begriff "wirkstoffenthaltende Zusammensetzung" oder "Beschichtungslösung" wie hierin verwendet, wird das Gemisch aus mindestens einem Wirkstoff, einer Trägersubstanz und einem Lösungsmittel oder Lösungsmittelgemisch verstanden, also eine tatsächliche Lösung, aus einem Wirkstoff, einer Trägersubstanz und mindestens einem weiteren Bestandteil, ausgewählt aus den hierin genannten Lösungsmitteln. Der Beschichtung kann ein Hilfsstoff zugesetzt sein. Dies kann ein Kristallisationsinhibitor oder eine Substanz sein die die Auflösung der Beschichtung bei der Dilatation weiter beschleunigt, Fettsäureester, Aminosäuren, Vitamine, Salze und/oder membranbildendende oder membrandisruptive Substanzen. Der Begriff "Lösung" soll ferner verdeutlichen, dass es sich um ein flüssiges Gemisch handelt, welches jedoch bereits eine gummiartige, gelartige, zähflüssige bzw. pastöse (dickviskose bzw. hochviskose) Konsistenz haben kann.

Es handelt sich bei der Beschichtungslösung nicht zwingend um eine streichfeste Lösung. Diese kann bei Verwendung einer Beschichtungslösung erst nach dem Auftragen und nach dem Entfernen des Lösungsmittels z.B. durch Trocknung erhalten werden.

Im Schmelzverfahren sind streichfeste Lösungen häufig nur durch schnelles Abkühlen oder Schockgefrieren einer Schmelze aus Wirkstoff und Träger zu erhalten. Es ist auch möglich die Ballonkatheter durch Aufbringen einer Schmelze aus mindestens einem antirestenotischen Wirkstoff und einem Träger zu beschichten. In diesem Fall ist kein zusätzliches Lösungsmittel anwesend.

Ferner sind die erfindungsgemäß beschichteten Katheterballons ohne gekrimpten Stent insbesondere zur Behandlung kleiner Gefäße, vorzugsweise kleiner Blutgefäße geeignet. Als kleine Gefäße werden solche mit einem Gefäßdurchmesser kleiner als 2,5 mm, vorzugsweise kleiner als 2,2 mm bezeichnet.

Ferner sind die erfindungsgemäß beschichteten Katheterballons ohne gekrimpten Stent insbesondere zur Behandlung peripherer Blutgefäße geeignet, bei denen sich die Implantation eines Stent als problematisch erwiesen hat.

Die folgenden Beispiele stellen mögliche Ausführungsformen der vorliegenden Erfindung dar, ohne jedoch den Schutzumfang auf diese konkreten Beispiele beschränken zu wollen.

### Beispiele

### Beispiel 1: Herstellung einer Beschichtungslösung aus Paclitaxel (Ptx) und Dexpanthenol

Hierzu wird 120 mg Paclitaxel (Sigma-Aldrich Chemie Gmbh, Deutschland) in 1000 µL Aceton gelöst. Desgleichen werden 30 mg Dexpanthenol (Carl Roth GmbH, Deutschland) in 500 µL Ethanol gelöst. Anschliessend werden beide Lösungen vereint.

### Beispiel 2A: Herstellung einer Beschichtungslösung aus Paclitaxel und PEG (1:2:2 w:w:w)

Hierzu werden 100 mg Paclitaxel und 200 mg PEG A und 200mg PEG B in 1000 µL Aceton gelöst.

PEG A ist PEG 400 (Sigma-Aldrich Chemie Gmbh, Deutschland) und hat eine Molekülmasse zwischen 380 und 420 und eine mittlere Molekülmasse Mₙ von 400.

PEG B ist ein Polyethylenglycol (Sigma-Aldrich Chemie Gmbh, Deutschland) mit einer Molekülmasse zwischen 950 und 1050.

### Beispiel 2B: Herstellung einer Beschichtungslösung aus Sirolimus und PEG (1:2:2 w:w:w)

Hierzu werden 100 mg Sirolimus (Merck4Biosciences, Deutschland) in Ethylacetat und 200 mg PEG A und 200mg PEG B in 1000 µL abs.Ethanol gelöst. Die beiden Lösungen werden vereinigt.

### Beispiel 3: Vollständige Beschichtung eines Faltenballons mit einer Beschichtungslösung aus PEG/Paclitaxel gemäß Beispiel 2A

Der Faltenballon wird in horizontaler Lage auf einer drehbaren Achse fixiert und mit der wirkstoffenthaltenden Lösung besprüht. Das Lösungsmittel wird anschliessend im Trockenschrank bei 70°C in 30 min verdampft.

Anschliessend lässt sich, wenn gewünscht, ein beschichteter oder unbeschichteter (bare stent) Stent aufbringen. Ebenso besteht die Möglichkeit einen hämokompatibel beschichteten Stent auf dem bereits beschichteten Ballonkatheter mit einem Coating der Wahl mittels Pipettierverfahren oder Sprühverfahren zu beschichten.

### Beispiel 4 : Beschichtung eines Faltenballons mit einer Beschichtungslösung aus Ptx/Dexpanthenol gemäß Beispiel 1 in den Falten des Ballons

Der Faltenballon wird in horizontaler Lage auf einer drehbaren Achse fixiert, so dass die zu befüllende Falte immer auf der oberen Seite zu liegen kommt. So wird Schritt für Schritt jede Falte mit der viskosen wirkstoffenthaltenden Lösung aus Beispiel 1 mit einer vom Faltenanfang zum Faltenende langsam durchgeführten Teflonkanüle als Erweiterung einer Nadelspritze befüllt und anschliessend im Trockenschrank bei 70°C in 30 min getrocknet. Es wird ein Faltenballon hergestelltet, bei dem ausschliesslich die Falten mit einer transparenten, streichfesten Lösung beschichtet sind. Besonders gut läßt sich dies nach einer Dilatation erkennen. Zur Kontrolle kann eine geringe Menge Farbstoff wie z.B. Curcumin beigefügt werden.

Anschliessend lässt sich, wenn gewünscht, ein beschichteter oder unbeschichteter (bare stent) Stent aufbringen. Ebenso besteht die Möglichkeit einen hämokompatibel beschichteten Stent auf dem bereits beschichteten Ballonkatheter mit einem Coating der Wahl mittels Pipettierverfahren oder Sprühverfahren zu beschichten.

### Beispiel 5: Herstellung einer Beschichtungslösung aus Paclitaxel (Ptx) und Vanillin

Hierzu wird 100 mg Paclitaxel in 1000 µL Aceton gelöst. Desgleichen werden 100 mg Vanillin in 500 µL Ethanol gelöst. Anschliessend werden beide Lösungen vereint.

### Beispiel 6 : Vollständige Beschichtung eines Stents mit einer Beschichtungslösung aus Vanillin/Paclitaxel

Der Stent wird zunächst auf einen Faltenballon aufgebracht. Der Faltenballon mit Stent wird in horizontaler Lage auf einer drehbaren Achse fixiert und mit der wirkstoffenthaltenden Lösung besprüht. Das Lösungsmittel wird anschliessend im Trockenschrank bei 80°C in 30 min verdampft.

### Beispiel 7: Herstellung einer Beschichtungslösung aus Rapamycin, Glycerin und Carrageenan.

Hierzu wird 100 mg Rapamycin in 500 µL Ethanol gelöst und 500 µL Glycerin hinzugefügt. Darin werden 5 mg Carrageenan zugesetzt.

### Beispiel 8: Vollständige Beschichtung eines Faltenballons mit einer Beschichtungslösung aus Rapamycin, Glycerin und Carrageenan

Der Faltenballon wird in horizontaler Lage auf einer drehbaren Achse fixiert, leicht dilatiert und mit der wirkstoffenthaltenden Lösung bepinselt. Das Lösungsmittel wird anschliessend im Trockenschrank bei 60°C in 20 min verdampft. Der Ballon wird danach wieder in Falten gelegt. Anschliessend lässt sich, wenn gewünscht, ein beschichteter oder unbeschichteter Stent aufbringen.

### Beispiel 9: Herstellung einer Beschichtungslösung aus Paclitaxel (Ptx), Carbopol^{®}980 und Mastix

Hierzu wird 100 mg Paclitaxel in 1000 µL Ethanol gelöst. Diese Lösung wird mit 8 mg geschmolzenem Chios-Mastix (Sigma-Aldrich Chemie Gmbh, Deutschland) (erhitzt auf circa 80°C) vermischt. Desgleichen werden 20 mg Carbopol in 500 µL Wasser gelöst. Anschliessend werden beide Lösungen vereint.

### Beispiel 10: Vollständige Beschichtung eines Faltenballons mit einer Beschichtungslösung aus Paclitaxel (Ptx), Carbopol^{®}980 und Mastix

Der Faltenballon wird in horizontaler Lage auf einer drehbaren Achse fixiert und mit der wirkstoffenthaltenden Lösung aus Beispiel 9 besprüht. Das Lösungsmittel wird anschliessend im Trockenschrank bei 70°C in 30 min verdampft.

Anschliessend lässt sich, wenn gewünscht, ein beschichteter oder unbeschichteter (bare stent) Stent aufbringen. Ebenso besteht die Möglichkeit einen hämokompatibel beschichteten Stent auf dem bereits beschichteten Ballonkatheter mit einem Coating der Wahl mittels Pipettierverfahren oder Sprühverfahren zu beschichten.

### Beispiel 11: Herstellung einer Beschichtungslösung aus Paclitaxel (Ptx), Dexpanthenol und Ricinolsäure

Hierzu wird 120 mg Paclitaxel in 1000 µL Aceton gelöst. Desgleichen werden 30 mg Dexpanthenol in 500 µL Ethanol gelöst. Anschliessend werden beide Lösungen vereint und 10 mg Ricinolsäure zugegeben.

### Beispiel 12: Beschichtung eines Faltenballons mit einer Beschichtungslösung aus Ptx/Dexpanthenol und Ricinolsäure in den Falten eines Ballons

Der Faltenballon wird in horizontaler Lage auf einer drehbaren Achse fixiert, so dass die zu befüllende Falte immer auf der oberen Seite zu liegen kommt. So wird Schritt für Schritt jede Falte mit der viskosen wirkstoffenthaltenden Lösung aus Beispiel 11 mit einer vom Faltenanfang zum Faltenende langsam durchgeführten Teflonkanüle als Erweiterung einer Nadelspritze befüllt und anschliessend im Trockenschrank bei 70°C in 30 min getrocknet.

Anschliessend lässt sich, wenn gewünscht, ein beschichteter oder unbeschichteter (bare stent) Stent aufbringen. Ebenso besteht die Möglichkeit einen hämokompatibel beschichteten Stent auf dem bereits beschichteten Ballonkatheter mit einem Coating der Wahl mittels Pipettierverfahren oder Sprühverfahren zu beschichten.

### Beispiel 13: Herstellung einer Beschichtungslösung aus Paclitaxel (Ptx), Polyethylenglykol und Resorcin

Hierzu wird 120 mg Paclitaxel in 800 µL Ethanol gelöst und 2 mg Resorcin (Merck4Biosciences, Deutschland) zugegeben. Desgleichen werden 200 mg PEG B (s. Beispiel 2) in 1000 µL Aceton. Anschliessend werden beide Lösungen vereint.

### Beispiel 14: Beschichtung eines Ballonkatheters mit einer Beschichtungslösung aus Paclitaxel (Ptx), Polyethylenglykol und Resorcin

Der Ballonkatheter wird über einen Adapter an die Antriebswelle des Rotationsmotor aufgesteckt und so fixiert, dass er in der Horizontalen ohne Durchknicken zu liegen kommt. Nachdem leichter Unterdruck auf den Ballon gezogen worden ist, wird die gesamte Ballonoberfläche mit der eingestellten Anzahl an Ballonüberstreichungen mit der Lösung beschichtet. Durch die Dosiernadel und den angeschweißten Schleppdraht wird ein Tropfen Lösung über den rotierenden Ballon geschleppt bis das Lösungsmittel so weit verdunstet ist, dass sich eine streichfeste Beschichtung gebildet hat. Anschließend wird der Katheter von der Maschine genommen und bei 60°C und weiterer Rotation über Nacht getrocknet.

Anschliessend lässt sich, wenn gewünscht, ein beschichteter oder unbeschichteter (bare stent) Stent aufbringen. Ebenso besteht die Möglichkeit einen hämokompatibel beschichteten Stent auf dem bereits beschichteten Ballonkatheter mit einem Coating der Wahl mittels Pipettierverfahren oder Sprühverfahren zu beschichten.

### Beispiel 15: Herstellung einer Beschichtungslösung aus Rapamycin, Dexpanthenol und Isomalt

Hierzu wird 150 mg Rapamycin (Merck4Biosciences, Deutschland) in 1000 µL Ethylacetat. Desgleichen werden 60 mg Dexpanthenol in 500 µL Ethanol gelöst. Anschliessend werden beide Lösungen vereint und 5 mg Isomalt (Carl Roth GmbH, Deutschland) zugegeben.

### Beispiel 16: Beschichtung eines Stents mit einer Beschichtungslösung aus Rapamycin/Dexpanthenol und Isomalt in den Falten eines Ballons

Nach dem Reinigen der Stents wird eine erste Schicht der Beschichtungslösung aus Beispiel 15 auf den Stent aufgesprüht. Nach dem Trocknen dieser Schicht bei Raumtemperatur wird die zweite Schicht der Beschichtungslösung aus Bespiel 15 aufgesprüht und anschliessend wird der Stent im Trockenschrank bei 70°C in 30 min getrocknet.

Anschliessend lässt sich, wenn gewünscht, ein beschichteter oder unbeschichteter (bare stent) Stent aufbringen. Ebenso besteht die Möglichkeit einen hämokompatibel beschichteten Stent auf dem bereits beschichteten Ballonkatheter mit einem Coating der Wahl mittels Pipettierverfahren oder Sprühverfahren zu beschichten.

### Beispiel 17: Untersuchung zur Haftfähigkeit einer Beschichtung

Hierzu wird einem Schwein ein Blutgefäß entnommen und in physiologischer Kochsalzlösung gelagert, welche auch durch das Gefäß perfundieren soll. Anschließend wird der Ballonkatheter in dem Gefäß expandiert und die anhaftende als auch die auf dem Ballonkatheter verbleibende Beschichtungsmenge ermittelt. Es hat sich gezeigt, dass alle in den Beispielen hergestellten Katheterballon nach diesem Test nur noch geringe Mengen (< 15%) der Beschichtung auf der Ballonoberfläche tragen.

### Beispiel 18:

### Kovalente hemokompatible Beschichtung von Stents:

Nicht expandierte gereinigte Stents aus medizinischem Edelstahl LVM 316 werden für 5 Minuten in eine 2%ige Lösung von 3 Aminopropyltriethoxysilan in einem Gemisch Ethanol/Wasser (50/50 : (v/v)) getaucht und anschließend getrocknet. Anschließend wurden die Stents über Nacht mit demineralisiertem Wasser gewaschen.

3 mg desulfatiertes und reacetyliertes Heparin wird in 30 ml 0,1M MES Puffer (2 (N Morpholino)ethansulfonsäure) pH 4,75 gelöst und mit 30 mg N Cyclohexyl-N' (2 morpholinoethyl)carbodiimid-methyl p toluolsulfonat versetzt. In dieser Lösung werden die Stents über Nacht bei 4°C gerührt. Anschließend wird mit Wasser und 4M NaCl-Lösung ausgiebig gespült.

## Patentansprüche

1. Kurzzeitimplanat und/oder Langzeitimplanat, wobei die Oberfläche des Kurzzeitimplanates und/oder Langzeitimplanates vollständig oder teilweise mit einer streichfesten Lösung in Form einer unterkühlten Schmelze enthaltend mindestens einen molekulardispers verteilten Wirkstoff und mindestens eine Trägersubstanz beschichtet ist, wobei die streichfeste Lösung auf der Oberfläche des Kurzzeitimplanates und/oder Langzeitimplanates in Form einer unterkühlten Schmelze optional einen Kristallisationsinhibitor enthält.

2. Kurzzeitimplanat und/oder Langzeitimplanat gemäß Anspruch 1, wobei das Mengenverhältnis des mindestens einen Wirkstoffs und der mindestens einen Trägersubstanz von 90 Gew.-% Wirkstoff zu 10 Gew.-% der Trägersubstanz bis 10 Gew.-% Wirkstoff zu 90 Gew.-% der Trägersubstanz beträgt.

3. Kurzzeitimplanat und/oder Langzeitimplanat gemäß einem der Ansprüche 1 oder 2, wobei die mindestens eine Trägersubstanz ausgewählt wird aus der Gruppe umfassend oder bestehend aus Polyether, Polylactonsäure, Polyethylenglykol, Poly(N-vinyl)-Pyrrolidon, N-Dodecyl-pyrrolidon, N-Decyl-pyrrolidon, N-Octyl-pyrrolidon, Polyvinylalkohole, Derivate von Polyvinylalkoholen, glycolierte Polyester, Polyphosphoester, Polyethylenoxidpropylenoxid, Polyethylenoxid, Hyaluronsäure, Copolymere mit PEG und Polypropylenglycol, Lipide, Phospholipide, Polyacrylsäure, Polyacrylate, Carboxymethylchitosane, Lanolin, Vanillin, Sorbit, Gelatine, Derivate von Gelatine, Fettsäurepartialglyceride mit einem Monoanteil von 50 bis 95 Gew.-%, hochdisperses Siliciumdioxid, Cellulose, Derivat von Cellulose, Hydroxypropylcellulose, Ethylcellulose, Stärke, Derivate von Stärke, Dextrine, Dextrane, α-Cyclodextrin, β-Cyclodextrin, γ-Cyclodextrin, Dimethyl-β-cyclodextrin, 2-Hydroxypropyl-β-cyclodextrin, Sterine, Cholesterol, Gallensäuren, Cholsäure, Lithocholsäure, N-Alkyllactame, N-Dodecylcaprolactam, N-Decyl-caprolactam, N-Octylcaprolactam, N-Dodecylvalerolactam, N-Decyl-valerolactam, N-Octylvalerolactam, Polyoxyethylen-Sorbitan-Monolaurate, Polyoxyethylen-Sorbitan-Monopalmitate, Polyoxyethylen-Sorbitan-Monostearat, Sorbitan-Monolaurat, Sorbitan-Monopalmitat, Sorbitan-Monostearat, Polyoxyethylen-oleylether, Polyoxyethylen-oleylether, Bis-[alpha-methyl-(4-methyl-benzyl)]-phenyl-polyglykolether, Bis-[alpha-methyl-(4-n-dodecyl)]-phenyl-polyglykolether, Bis-(4-methyl-benzyl)-phenyl-polyglykolether, Bis-(4-n-dodecyl-benzyl)-phenyl-polyglykolether, Tris-[alpha-methyl-(4-methyl-benzyl)]-phenyl-polyglykolether, Nonylphenol-polyglykolether und Nonylphenol-diglykolether.

4. Kurzzeitimplanat und/oder Langzeitimplanat gemäß Anspruch 3, wobei es sich bei der mindestens einen Trägersubstanz um Dexpanthenol, Hyaluronsäure, Vanillin, Carboxymethylchitosan oder PEG handelt.

5. Kurzzeitimplanat und/oder Langzeitimplanat gemäß einem der Ansprüche 1 - 4, wobei das Kurzzeitimplanat und/oder Langzeitimplanat ein Katheterballon mit oder ohne Stent ist.

6. Katheterballon gemäß Anspruch 5, wobei der mindestens eine Wirkstoff antirestenotisch ist und ausgewählt wird aus der Gruppe umfassend oder bestehend aus: Paclitaxel, Docetaxel, Sirolimus, Biolimus A9, Zotarolimus, Everolimus, Myolimus, Novolimus, Pimecrolimus, Tacrolimus, Ridaforolimus, Temsirolimus, Cyclosporine, Vitamin K, Propofol und Diazepam.

7. Katheterballon gemäß Anspruch 6, wobei es sich bei dem mindestens einen antirestenotischen Wirkstoff um Paclitaxel handelt.

8. Katheterballon gemäß Anspruch 6, wobei es sich bei dem mindestens einen antirestenotischen Wirkstoff um Sirolimus handelt.

## Claims

1. Short-term implant and/or long-term implant, wherein the surface of the short-term implant and/or long-term implant is coated completely or partially with a stably spreadable solution in form of an undercooled melt containing at least one molecular-disperse distributed active substance and at least one carrier, wherein the stably spreadable solution on the surface of the short-term implant and/or long-term implant in form of an undercooled melt optionally contains a crystallization inhibitor.

2. Short-term implant and/or long-term implant according to claim 1, wherein the proportion of the at least one active substance and the at least one carrier ranges from 90% by weight of the active substance to 10% by weight of the carrier to 10% by weight of the active substance to 90% by weight of the carrier.

3. Short-term implant and/or long-term implant according to one of the claims 1 or 2, wherein the at least one carrier is selected from the group comprising or consisting of polyether, polylactonic acid, polyethylene glycol, poly(N-vinyl) pyrrolidone, N-dodecyl pyrrolidone, N-decyl pyrrolidone, N-octyl pyrrolidone, polyvinyl alcohols, derivatives of polyvinyl alcohols, glycolated polyesters, polyphosphoesters, polyethylene oxide propylene oxide, polyethylene oxide, hyaluronic acid, copolymers with PEG and polypropylene glycol, lipids, phospholipids, polyacrylic acid, polyacrylates, carboxymethyl chitosanes, lanolin, vanillin, sorbitol, gelatine, derivatives of gelatine, fatty acid partial glycerides with a monocontent of 50 to 95 w.-%, highly dispers silicon dioxide, cellulose, derivative of cellulose, hydroxypropyl cellulose, ethyl cellulose, starch, derivatives of starch, dextrines, dextranes, α-cyclodextrin, β-cyclodextrin, γ-cyclodextrin, dimethyl-β-cyclodextrin, 2-hydroxypropyl-β-cyclodextrin, steroles, cholesterol, bile acids, cholic acid, lithocholic acid, N-alkyl lactames, N-dodecyl caprolactam, N-decyl caprolactam, N-octyl caprolactam, N-dodecyl valerolactam, N-decyl valerolactam, N-octyl valerolactam, polyoxyethylene sorbitan monolaurates, polyoxyethylene sorbitan monopalmitates, polyoxyethylene sorbitan monostearate, sorbitan monolaurate, sorbitan monopalmitate, sorbitan monostearate, polyoxyethylene oleyl ether, bis-[alpha-methyl-(4-methyl-benzyl)]-phenyl-polyglycol ether, bis-[alpha-methyl-(4-n-dodecyl)]-phenyl-polyglycol ether, bis-(4-methyl-benzyl)-phenyl-polyglycol ether, bis-(4-n-dodecyl-benzyl)-phenyl-polyglycol ether, tris-[alpha-methyl-(4-methyl-benzyl)]-phenyl-polyglycol ether, nonylphenol polyglycol ether and nonylphenol diglycol ether.

4. Short-term implant and/or long-term implant according to claim 3, wherein the at least one carrier is dexpanthenol, hyaluronic acid, vanillin, carboxymethyl chitosan or PEG.

5. Short-term implant and/or long-term implant according to one of the claims 1 - 4, wherein the short-term implant and/or long-term implant is a catheter balloon with or without a stent.

6. Catheter balloon according to claim 5, wherein the at least one active substance is anti-restenotic and is selected from the group comprising or consisting of: paclitaxel, docetaxel, sirolimus, biolimus A9, zotarolimus, everolimus, myolimus, novolimus, pimecrolimus, tacrolimus, ridaforolimus, temsirolimus, cyclosporine, vitamin K, propofol and diazepam.

7. Catheter balloon according to claim 6, wherein the at least one anti-restenotic active substance is paclitaxel.

8. Catheter balloon according to claim 6, wherein the at least one anti-restenotic active substance is sirolimus.

## Revendications

1. Un implant à court terme et/ou un implant à long terme, dans lequel la surface de l'implant à court terme et/ou de l'implant à long terme est complètement ou partiellement revêtue d'une solution non-coulante sous forme de liquide en surfusion contenant au moins une substance active distribuée en dispersion moléculaire et au moins un véhicule, où la solution non-coulante sur la surface de l'implant à court terme et/ou de l'implant à long terme sous forme de liquide en surfusion contient optionnellement un inhibiteur de cristallisation.

2. L'implant à court terme et/ou l'implant à long terme selon la revendication 1, dans lequel le ratio entre l'au moins une substance active et l'au moins un véhicule est de 90% en poids de substance active à 10% en poids de véhicule à 10% en poids de substance active à 90% en poids de véhicule.

3. L'implant à court terme et/ou l'implant à long terme selon une des revendications 1 et 2, dans lequel l'au moins un véhicule est choisi parmi le groupe comprenant ou consistant en : polyéther, acide polylactonique, polyéthylène glycol, poly(N-vinyl)-pyrrolidone, N-dodécyl-pyrrolidone, N-décyl-pyrrolidone, N-octyl-pyrrolidone, alcools polyvinyliques, dérivés d'alcool polyvinylique, polyester glycolé, polyphosphoesters, poly(éthylène oxyde propylène oxyde), poly(éthylène oxyde), acide hyaluronique, copolymères de PEG et polypropylène glycol, lipides, phospholipides, acide polyacrylique, polyacrylates, carboxyméthyl-chitosanes, lanoline, vanilline, sorbitol, gélatine, dérivés de gélatine, glycérides partiels d'acides gras avec une teneur en mono-glycérides de 50 jusqu'à 95% en poids, dioxyde de silicium hautement dispersé, cellulose, dérivés de cellulose, hydroxypropyl-cellulose, éthyl-cellulose, amidon, dérivés d'amidon, dextrines, dextranes, α-cyclodextrine, β-cyclodextrine, γ-cyclodextrine, diméthyl-β-cyclodextrine, 2-hydroxypropyl-β-cyclodextrine, stérols, cholestérol, acides biliaires, acide cholique, acide litocholique, N-alkyl-lactames, N-dodécyl-caprolactame, N-décyl-caprolactame, N-octyl-caprolactame, N-dodécyl-valérolactame, N-décyl-valérolactame, N-octyl-valérolactame, polyoxyéthylène sorbitan monolaurates, polyéthoxylène sorbitan monopalmitate, polyéthylène sorbitan monostéarate, sorbitan monolaurate, sorbitan monopalmitate, sorbitan monostéarate, polyoxyéthylène oléyl éther, bis-[alpha-méthyl-(4-méthyl-benzyl)]-phényl-polyglycol éther, bis-[alpha-méthyl-(4-n-dodécyl)]-phényl-polyglycol éther, bis-(4-méthyl-benzyl)-phényl-polyglycol éther, bis-(4-n-dodécyl-benzyl)-phényl-polyglycol éther, tris-[alpha-méthyl-(4-méthyl-benzyl)]-phényl-polyglycol éther, nonylphénol-polyglycol éther et nonylphénol-diglycol éther.

4. L'implant à court terme et/ou l'implant à long terme selon la revendication 3, dans lequel l'au moins un véhicule est acide hyaluronique, vanilline, carboxyméthyl chitosane ou PEG.

5. L'implant à court terme et/ou l'implant à long terme selon une des revendications 1 - 4, où l'implant à court terme et/ou l'implant à long terme est un ballonnet à cathéter avec un sans stent.

6. Le ballonnet à cathéter selon la revendication 5, dans lequel l'au moins au moins une substance active est anti-sténotique et est choisie parmi le groupe comprenant ou consistant en: paclitaxel, docetaxel, sirolimus, biolimus A9, zotarolimus, everolimus, myolimus, novolimus, pimecrolimus, tacrolimus, ridaforolimus, temsirolimus, ciclosporine, vitamine K, propofol et diazépam.

7. Le ballonnet à cathéter selon la revendication 6, dans lequel l'au moins une substance active anti-sténotique est paclitaxel.

8. Le ballonnet à cathéter selon la revendication 6, dans lequel l'au moins une substance active anti-sténotique est sirolimus.
